# FASCICULE DE BREVET EUROPEEN

(11) **EP 3 027 231 B1**
(45) Date de publication et mention de la délivrance du brevet: **30.08.2017**
(21) Numéro de dépôt: 14790152.4
(22) Date de dépôt: 22.07.2014
(51) Int. Cl.: A61L 9/12

(54) **DISPOSITIF DE DIFFUSION DE FRAGRANCE TELLE QU'UN PARFUM**
VORRICHTUNG ZUR DIFFUSION VON DUFTSTOFFEN WIE Z. B. PARFÜMEN
DEVICE FOR DIFFUSING FRAGRANCE, SUCH AS A PERFUME

(30) Priorité: 01.08.2013 FR 1357666
(43) Date de publication de la demande: 08.06.2016
(73) Titulaire: Techniplast, 27400 Louviers (FR)
(72) Inventeur: SIMIAN, Frédéric, F-27430 Saint Etienne du Vauvray (FR); LAMBOUX, Jean-Philippe, F-27370 Saint Didier des Bois (FR)
(74) Mandataire: Petit, Maxime
(86) Numéro de dépôt international: PCT/FR2014/051890
(87) Numéro de publication internationale: WO 2015/015090

(56) Documents cités:
- EP-A1- 2 336 079
- EP-A1- 2 500 277
- WO-A1-2006/113254
- WO-A1-2011/128604

## Description

L'invention concerne un dispositif de diffusion d'une fragrance telle qu'un parfum.

On connait du document FR 2 958 854 un diffuseur de fragrance comprenant :
- deux bouchons verseurs montés tête-bêche et présentant chacun un tube d'écoulement,
- une plaquette de matière poreuse montée entre les deux bouchons verseurs,
- une bague extérieure pourvue, en regard de la plaquette, d'au moins un orifice de diffusion de la fragrance.

Chaque bouchon verseur comprend également un trou d'écoulement qui permet le passage de la fragrance dans la plaquette de matière poreuse, puis vers l'extérieur du dispositif. La fragrance est par exemple contenue dans un flacon disposé au dessus du diffuseur.

Bien que satisfaisant ce diffuseur pourrait toutefois être simplifié.

A cet effet, l'invention vise, selon un premier aspect, un dispositif de diffusion d'une fragrance, caractérisé en ce qu'il comprend :
- deux parois en regard espacées l'une de l'autre suivant un axe longitudinal de manière à définir entre elles une cavité qui s'étend transversalement jusqu'à une paroi périphérique pourvue d'au moins une ouverture pour la diffusion de fragrance à l'extérieur du dispositif, ladite au moins une ouverture mettant en communication la cavité avec l'extérieur du dispositif,
- au moins une mèche en matière poreuse qui s'étend dans la cavité suivant l'axe longitudinal en traversant les deux parois par l'intermédiaire de ses deux extrémités opposées respectives qui font saillie chacune dans une zone externe adjacente à la paroi concernée,
- au moins un orifice de reprise d'air qui traverse chaque paroi de manière à mettre en communication chaque zone externe adjacente à chaque paroi avec la cavité et donc avec l'extérieur du dispositif.
Dans le dispositif selon l'invention ladite au moins une mèche s'imprègne de fragrance et assure ainsi à la fois la diffusion de fragrance (située au dessus du dispositif, notamment dans un flacon supérieur) dans la cavité et à l'extérieur, via la portion centrale de ladite au moins une mèche qui s'étend dans la cavité, et le goutte à goutte en dessous du dispositif (notamment dans un flacon inférieur).

Le dispositif selon l'invention est donc plus simple que celui de l'art antérieur dans lequel les fonctions de diffusion et de goutte à goutte sont dissociées.

Par ailleurs, dans l'art antérieur précité pour assurer le goutte à goutte de très petits trous (ex : 0,2 à 0,5 mm) sont nécessaires et ceux-ci ont tendance à se boucher en présence des produits gras qui rentrent dans la composition des fragrances (parfums). Ce problème ne se pose pas avec le dispositif selon l'invention dans lequel le goutte à goutte est assuré par ladite au moins une mèche poreuse.

Contrairement au dispositif de l'art antérieur précité, l'air de reprise qui compense le volume de fragrance s'écoulant à travers ladite au moins une mèche vient de l'extérieur du dispositif. Ceci permet de dissocier la compensation d'air dans le flacon qui dispense sa fragrance de l'écoulement de cette fragrance et, donc, du goutte à goutte. Dans l'art antérieur, les flacons communiquent entre eux par des tubes d'écoulement et de passage d'air. Ainsi, la pression ou la dépression de l'un ou l'autre des flacons influe sur le fonctionnement du goutte à goutte (par exemple, lorsqu'un équilibre de pressions doit s'établir), ce qui n'est pas le cas dispositif selon l'invention.

Selon d'autres caractéristiques possibles, prises isolément ou en combinaison l'une avec l'autre :
- le dispositif comprend, de part et d'autre des deux parois, dans chaque zone externe adjacente à chaque paroi, des moyens de fixation destinés chacun à fixer au dispositif un col d'un flacon ;
- chaque paroi présente une forme générale concave dont la concavité est orientée vers la cavité et comporte une région centrale ;
- ledit au moins un orifice est situé à la périphérie de la région centrale ;
- plusieurs orifices de reprise d'air traversants sont pratiqués dans chaque paroi, le dispositif comprenant des moyens d'obturation qui, selon leur position, sont aptes à obturer tout ou partie desdits orifices ;
- le dispositif comprend une pluralité de mèches poreuses réparties dans la cavité ;
- le dispositif comprend un système de clapet à bille aménagé dans chaque paroi au droit d'un orifice traversant la paroi de manière à ce que la bille du système de clapet à bille de la paroi supérieure obture l'orifice correspondant et que la bille du système de clapet à bille de la paroi inférieure laisse dégagé l'orifice correspondant ;
- l'axe longitudinal est un axe vertical du dispositif.

Selon un deuxième aspect, l'invention vise également un système de diffusion d'une fragrance, caractérisé en ce qu'il comprend :
- un dispositif de diffusion tel que brièvement exposé ci-dessus,
- un premier flacon supérieur ayant un premier col dont l'ouverture est disposée en vis-à-vis d'une des deux parois en regard dite paroi supérieure, un second flacon inférieur ayant un deuxième col dont l'ouverture est disposée en vis-à-vis de l'autre paroi dite inférieure, le premier flacon supérieur comprenant la fragrance qui, par imprégnation de la mèche, provoque, d'une part, la diffusion de la fragrance dans la cavité et à l'extérieur du dispositif *via* ladite au moins une ouverture et, d'autre part, le goutte à goutte dans le second flacon inférieur.

Ce système présente les mêmes avantages et caractéristiques que ceux mentionnés en relation avec le dispositif de diffusion mentionné plus haut et ils ne seront donc pas répétés ici.

D'autres caractéristiques et avantages apparaîtront au cours de la description qui va suivre, donnée uniquement à titre d'exemple non limitatif et faite en référence aux dessins annexés, sur lesquels :
- la figure 1 est une vue générale schématique en coupe axiale d'un dispositif de diffusion d'une fragrance selon un premier mode de réalisation de l'invention ;
- la figure 2 illustre une variante de réalisation du dispositif de la figure 1 ;
- la figure 3 est une vue générale schématique en coupe axiale d'un dispositif de diffusion d'une fragrance selon un deuxième mode de réalisation de l'invention.

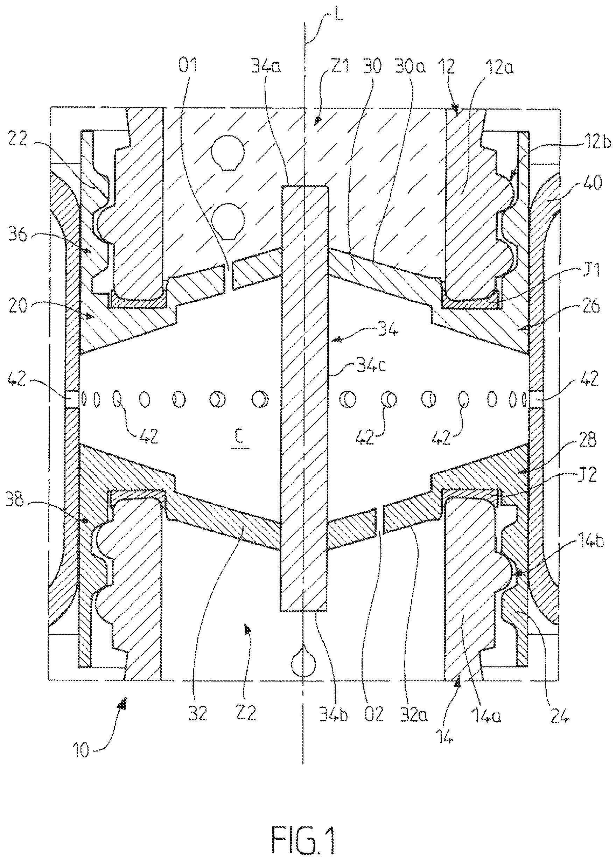

Comme représenté à la figure 1 et désigné par la référence générale notée 10, un système de diffusion d'une fragrance comprend un dispositif de diffusion d'une fragrance 20 et deux flacons 12, 14 dont seuls les cols respectifs 12a, 14a sont représentés.

Un premier flacon supérieur 12 contenant la fragrance (ex : un parfum d'ambiance) à diffuser est monté au dessus du dispositif 20 avec son col 12a orienté vers le bas.

Le dispositif 20 comprend des premiers moyens de fixation 22 qui sont destinés à fixer le col 12a audit dispositif. Ces moyens de fixation sont par exemple réalisés sous la forme d'un filetage interne pour le vissage avec le filetage externe 12b sur la surface externe du col 12a.

Un deuxième flacon inférieur 14 destiné à récupérer la fragrance non diffusée provenant du flacon supérieur 12 est monté sous le dispositif 20 avec son col 14a orienté vers le haut.

Le dispositif de diffusion 20 comprend des deuxièmes moyens de fixation 24 qui sont destinés à fixer le col 14a audit dispositif. Ces moyens de fixation sont par exemple réalisés sous la forme d'un filetage interne pour le vissage avec le filetage externe 14b sur la surface externe du col 14a. D'autres moyens de fixation tels que par encliquetage peuvent être envisagés.

Le système 10 et son dispositif 20 ainsi que les deux flacons sont agencés verticalement sur la figure 1 afin que la fragrance contenue dans le flacon supérieur 12 puisse s'écouler par gravité.

Le dispositif 20 comprend deux pièces, à savoir une pièce supérieure 26 et une pièce inférieure 28 disposées en regard et espacées l'une de l'autre suivant un axe longitudinal L qui, ici, est orienté verticalement.

Les deux pièces 26, 28 comprennent chacune une paroi 30, 32 disposée en vis-à-vis l'une de l'autre et qui définissent entre elles une cavité centrale C. Les parois 30, 32 sont perforées en leur milieu (région centrale) afin de permettre à une mèche poreuse 34 de s'étendre axialement dans la cavité C entre les parois, en étant maintenue dans celles-ci par l'intermédiaire de ses deux extrémités opposées respectives 34a, 34b. Ces deux extrémités opposées 34a, 34b traversent les parois respectives 30, 32 et font saillie chacune dans une zone externe au dispositif (Z1, Z2) et qui est adjacente à la paroi concernée.

Ainsi que représenté sur la figure 1, chaque zone externe est une zone interne au col d'un flacon.

La mèche est réalisée en matière poreuse de type connu.

Les parois 30, 32 s'étendent transversalement à partir de leur région centrale vers leur périphérie au niveau de laquelle s'étend axialement une paroi 36, 38. Les deux parois d'extension axiales 36, 38 des pièces 26, 28 s'étendent dans des directions opposées et en éloignement de la cavité C. Les moyens de fixation 22, 24 sont aménagés sur la surface intérieure de ces parois. On notera que le col 12a, 14a de chaque flacon, une fois fixé au dispositif 20, vient en butée contre un joint respectif J1, J2 aménagé dans un évidement de la face externe 30a, 32a de la paroi d'extension transversale considérée.

Comme représenté, chaque paroi transversale 30, 32 présente une forme générale concave dont la concavité est orientée vers la cavité C. Chaque paroi a plus particulièrement une forme d'entonnoir convergeant vers la région centrale.

Chaque pièce 26, 28 présente de façon générale une symétrie de révolution axiale à quelques détails de réalisation près. Ainsi, les parois d'extension axiale 36, 38 ont chacune une forme sensiblement cylindrique leur conférant une forme de jupe.

Le dispositif 20 comprend également à sa périphérie une paroi périphérique 40 ayant une forme générale de bague entourant l'ensemble formé des deux pièces 26, 28 en vis-à-vis et de la cavité centrale séparatrice C. Cette paroi périphérique 40 forme une pièce d'habillage et est fixée sur chacun des ensembles précités par exemple par collage, soudage ou par un autre moyen approprié.

La paroi périphérique 40 est munie d'au moins une ouverture 42 pour la diffusion de fragrance à l'extérieur du dispositif. Ladite au moins une ouverture 42 est pratiquée dans la région de la paroi qui est située autour de la cavité C de manière à mettre en communication cette cavité avec l'extérieur du dispositif. En pratique, pour une meilleure répartition de la diffusion de la fragrance, plusieurs ouvertures 42 sont disposées sur le pourtour de la paroi périphérique 40 comme représenté sur la figure 1, dans une région équatoriale du dispositif.

Chacune des parois d'extension transversale 30, 32 comporte au moins un orifice de reprise d'air O1, O2 qui la traverse dans son épaisseur de manière à mettre en communication chaque zone externe Z1, Z2 adjacente à chaque paroi avec la cavité centrale C et, donc, avec l'extérieur du dispositif par l'intermédiaire des ouvertures 42.

Dans la configuration de la figure 1 ledit au moins un orifice de reprise d'air est situé à la périphérie de la région centrale de chaque paroi, de manière décalée par rapport à la position centrale de la mèche 34.

On notera que plusieurs orifices peuvent être prévus dans chaque paroi 30, 32 afin d'augmenter la vitesse d'écoulement et donc du goutte à goutte. En effet, cette vitesse dépend notamment de la capacité du dispositif à reprendre de l'air.

Lors du fonctionnement du système 10 de diffusion d'une fragrance, le liquide (fragrance) présent dans le flacon supérieur 12 retourné imprègne l'extrémité supérieure 34a de la mèche 34 et s'écoule à l'intérieur de celle-ci, sur toute sa longueur. La mèche étant imprégnée de liquide diffuse radialement ou transversalement la fragrance à partir de sa partie centrale 34c qui est en contact avec l'air de la cavité C. La cavité C est une zone de diffusion dans laquelle la fragrance se diffuse jusqu'aux ouvertures 42 qu'elle traverse pour sortir du dispositif 20.

La mèche 34 crée en quelque sorte une aspiration du liquide du flacon 12 et donc une dépression dans ledit flacon (effet de pompage). Cette dépression est compensée par l'air externe qui entre dans le flacon par ledit au moins un orifice de reprise d'air O1.

Tout le liquide imprégnant la mèche n'est pas diffusé dans la cavité. En effet, une partie du liquide s'écoule le long de la mèche jusqu'à son extrémité inférieure 34b débouchante et s'écoule dans le flacon inférieur 14 sous la forme d'un goutte à goutte. Lorsque le liquide « non diffusé » est passé dans le flacon inférieur le phénomène de diffusion prend fin. La contenance du flacon inférieur, est dimensionnée pour que le niveau du liquide transféré dans le flacon soit situé en dessous de l'extrémité inférieure 34b de la mèche.

Lorsqu'il n'y a plus de liquide dans le flacon supérieur 12 et que le flacon inférieur 14 est rempli (au moins partiellement), le système est retourné : le flacon inférieur 14 devient le flacon supérieur et inversement, et le mode opératoire décrit ci-dessus se répète.

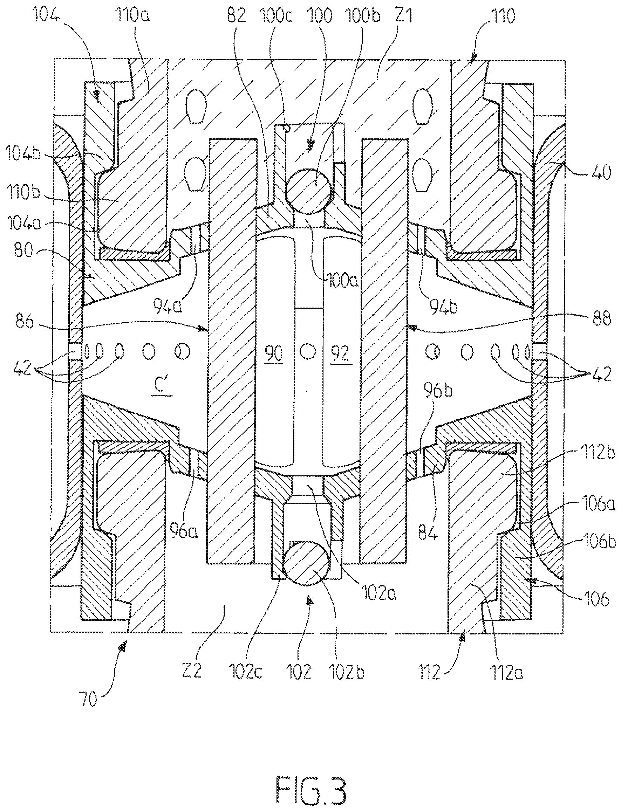

On notera que la vitesse d'écoulement du liquide dans la mèche et donc la vitesse d'écoulement du goutte à goutte dépend du diamètre du ou des orifices de reprise d'air O1, O2. Cette vitesse peut également dépendre de la longueur, de la densité et du diamètre de la mèche, voire du nombre de mèches lorsqu'il y en a plusieurs (figure 3).

Les parois 30 et 32 ont été rendues creuses ou concaves dans leur région centrale entourant la mèche afin que l'orifice ou les orifices 02 présents dans la paroi 32 (le même raisonnement s'applique avec l'orifice ou les orifices O1 présents dans la paroi 30 quand le système est retourné) soient disposés à une altitude supérieure à celle d'un point bas de la paroi entourant la mèche et autour duquel du liquide provenant d'un éventuel suintement pourrait s'accumuler. Un tel agencement réduit considérablement le risque que du liquide de suintement ne vienne obstruer le ou les orifices 02 lors du fonctionnement du système.

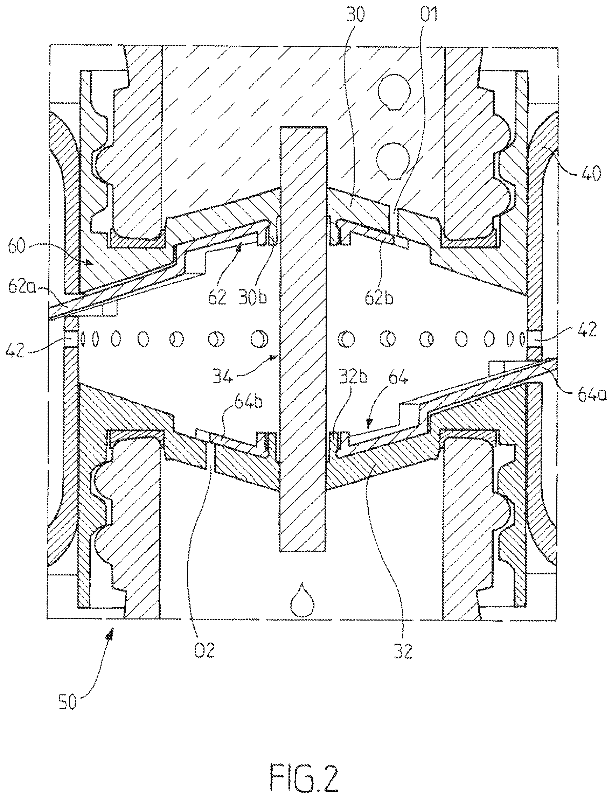

La figure 2 illustre une variante de réalisation du système de la figure 1. Le système 50 selon cette variante comprend un dispositif de diffusion 60 qui comporte des moyens de réglage de la reprise d'air à travers ledit au moins un orifice de reprise d'air O1 (resp. O2). Les autres éléments du système et du dispositif sont inchangés par rapport à la figure 1 et conservent donc les mêmes références.

Les moyens de réglage du dispositif 60 permettent de faire varier la vitesse d'écoulement du liquide dans la mèche et donc la vitesse de formation des gouttes.

Dans l'exemple illustré sur la figure 2 (la figure est une symétrie de type miroir par rapport à la figure 1) chaque paroi comporte plusieurs orifices de reprise d'air (O1 pour la paroi 30 et O2 pour la paroi 32) dont un seul est représenté. Plus particulièrement, le dispositif comporte des moyens de réglage associés à chaque série d'orifices O1 et O2 de chaque plaque 30, 32.

Par exemple, ces moyens prennent chacun la forme d'un organe d'obturation 62, 64 qui est monté rotatif autour d'un moyeu central (extension 30b, 32b) solidaire de la paroi associée 30, 32 (la mèche 34 est montée à l'intérieur de ces moyeux) et qui peut être actionné par une manette de commande 62a, 64a (manette de réglage) disposée à l'extérieur du dispositif. Des ouvertures supplémentaires, élargies par rapport aux ouvertures 42, ont été pratiquées dans la paroi périphérique 40 pour permettre le passage de la manette de commande associée. L'organe d'obturation comporte une extrémité d'obturation 62b, 64b qui, par rotation commandée à partir de la manette, vient obturer un ou plusieurs orifices de reprise d'air ou, au contraire, les libérer. L'organe d'obturation peut ainsi avoir une forme d'extrémité d'obturation adaptée afin d'obturer au mieux le ou les orifices. L'organe peut par exemple prendre la forme d'un excentrique.

Un système de diffusion 70 selon un deuxième mode de réalisation est représenté à la figure 3 et comprend un dispositif de diffusion 80.

Le dispositif 80 comprend toujours deux parois d'extension transversales 82, 84 en regard de l'une de l'autre et définissant entre elles une cavité centrale C' ou zone de diffusion.

Plusieurs mèches poreuses sont disposées dans la cavité C' et sont montées par leurs deux extrémités opposées traversantes dans les deux parois respectives 82, 84.

On dénombre par exemple quatre mèches dont deux, 86, 88, sont représentées au premier plan et deux, 90, 92, en arrière-plan.

Ces mèches sont réparties régulièrement autour de la région centrale de chaque paroi 82, 84 qui est creusée ou concave (fond de la paroi en forme générale d'entonnoir). Un nombre et un agencement différent de mèches peuvent être envisagés.

Plusieurs orifices de reprise d'air 94a, 94b et 96a, 96b sont prévus dans chaque paroi 82, 84. Deux orifices sont représentés pour chaque paroi. Toutefois, un nombre et un agencement différent d'orifices peuvent être envisagés.

Dans l'exemple de la figure 3, les orifices sont répartis à la périphérie extérieure des mèches. La région centrale de chaque paroi 82, 84 intègre un système de clapet à bille 100, 102 à l'aplomb d'un orifice traversant la paroi concernée et débouchant dans la cavité C.

Le système de clapet à bille 100 (resp. 102) comprend un orifice traversant 100a (resp. 102a) dont les bords supérieurs forment un siège de clapet et une bille 100b (resp. 102b) montée à l'intérieur d'un puits 100c (resp. 102c) qui s'étend axialement depuis les bords de l'orifice dans la zone Z1 (resp. Z2). Sous l'effet de la gravité la bille 100b repose sur son siège de clapet.

La bille 100b formant clapet obture l'orifice 100a pour empêcher que du liquide présent dans le col du flacon supérieur ne passe directement par cet orifice.

La bille 102b, quant à elle, repose sous l'effet de la gravité sur un épaulement aménagé sur le bord interne de l'extrémité libre du puits 102c, sans toutefois obstruer le puits.

Lorsque du liquide suinte dans la cavité, il s'écoule jusque dans l'orifice 102a et à l'intérieur du puits 102c, puis dans le flacon inférieur.

On notera que les parois d'extension axiale 104 et 106 du dispositif présentent des moyens de fixation qui diffèrent des moyens de fixation 22 et 24 de la figure 1.

En effet, les moyens de fixation comprennent, aménagés dans la paroi 104 (resp. 106) et du côté intérieur de celle-ci, au moins un évidement 104a (resp. 106a) placé en arrière d'au moins une portion de paroi plus épaisse 104b (resp. 106b). Cet évidement permet d'accueillir un ergot externe 110b aménagé sur le col 110a du flacon supérieur 110 et, ainsi, de retenir le col du flacon dans cette position immobilisée par rapport au dispositif 80.

On notera que ces moyens de fixation du dispositif comprennent plusieurs évidements et portions épaissies à la circonférence de la paroi 104 (resp. 106). Le col 110a (resp. 112a) est pourvu de plusieurs ergots externes répartis localement à la périphérie du col. Par un mouvement d'insertion axiale du col 110a (resp. 112a) à l'intérieur de la pièce axiale 104 (resp. 106) et de rotation, les ergots viennent se loger dans les évidements correspondants pour assurer le blocage axial du flacon correspondant.

Selon une variante non représentée, le col 110a (resp.112a) ne présente pas d'ergots externes mais un épaulement continu sur toute sa périphérie externe. Le col est introduit en force dans l'embout de fixation 104, 106 qui est en matériau déformable (ex : plastique).

Toutes les autres caractéristiques décrites en relation à la figure 1 sont reprises ici et procurent les mêmes fonctions et avantages.

## Revendications

1. Dispositif (20 ; 60 ; 80) de diffusion d'une fragrance comprenant
- deux parois (30 ; 32 ; 82 ; 84) en regard espacées l'une de l'autre suivant un axe longitudinal (L) de manière à définir entre elles une cavité (C ; C') qui s'étend transversalement jusqu'à une paroi périphérique (40) pourvue d'au moins une ouverture (42) pour la diffusion de fragrance à l'extérieur du dispositif, ladite au moins une ouverture mettant en communication la cavité avec l'extérieur du dispositif,
- au moins un orifice de reprise d'air (01, 02 ; 94a, 94b, 96a, 96b) qui traverse chaque paroi de manière à mettre en communication chaque zone externe adjacente à chaque paroi avec la cavité et donc avec l'extérieur du dispositif, **caractérisé en ce qu'**il comprend
- au moins une mèche (34 ; 86-92) en matière poreuse qui s'étend dans la cavité suivant l'axe longitudinal en traversant les deux parois par l'intermédiaire de ses deux extrémités opposées respectives (34a, 34b) qui font saillie chacune dans une zone externe (Z1, Z2) adjacente à la paroi concernée.

2. Dispositif selon la revendication 1, **caractérisé en ce qu'**il comprend, de part et d'autre des deux parois, dans chaque zone externe (Z1, Z2) adjacente à chaque paroi, des moyens de fixation (22, 24 ; 104a, 104b, 106a, 106b) destinés chacun à fixer au dispositif un col (12a, 12b; 110a, 110b) d'un flacon.

3. Dispositif selon la revendication 1 ou 2, **caractérisé en ce que** chaque paroi (30, 32 ; 82, 84) présente une forme générale concave dont la concavité est orientée vers la cavité et comporte une région centrale.

4. Dispositif selon l'une des revendications 1 à 3, **caractérisé en ce que** ledit au moins un orifice est situé à la périphérie de la région centrale.

5. Dispositif selon l'une des revendications 1 à 4, **caractérisé en ce que** plusieurs orifices de reprise d'air traversants (O1, O2) sont pratiqués dans chaque paroi, le dispositif comprenant des moyens d'obturation (62, 64) qui, selon leur position, sont aptes à obturer tout ou partie desdits orifices.

6. Dispositif selon l'une des revendications 1 à 5, **caractérisé en ce qu'**il comprend une pluralité de mèches poreuses (86-92) réparties dans la cavité.

7. Dispositif selon l'une des revendications 1 à 6, **caractérisé en ce qu'**il comprend un système de clapet à bille (100, 102) aménagé dans chaque paroi (82, 84) au droit d'un orifice traversant (100a, 102a) la paroi de manière à ce que la bille (100b, 102b) du système de clapet à bille de la paroi supérieure obture l'orifice correspondant et que la bille du système de clapet à bille de la paroi inférieure laisse dégagé l'orifice correspondant.

8. Dispositif selon l'une des revendications 1 à 7, **caractérisé en ce que** l'axe longitudinal (L) est un axe vertical du dispositif.

9. Système (10 ; 50 ; 70) de diffusion d'une fragrance, **caractérisé en ce qu'**il comprend :
- un dispositif de diffusion (20 ; 60 ; 80) selon l'une des revendications 1 à 8,
- un premier flacon supérieur (12 ; 110) ayant un premier col (12a ; 110a) dont l'ouverture est disposée en vis-à-vis d'une (30 ; 82) des deux parois en regard dite paroi supérieure, un second flacon inférieur (14 ; 112) ayant un deuxième col (14a ; 112a) dont l'ouverture est disposée en vis-à-vis de l'autre paroi dite inférieure (32 ; 84), le premier flacon supérieur comprenant la fragrance qui, par imprégnation de la mèche 34 ; 86-92), provoque, d'une part, la diffusion de la fragrance dans la cavité (C, C') et à l'extérieur du dispositif *via* ladite au moins une ouverture (42) et, d'autre part, le goutte à goutte dans le second flacon inférieur.

## Patentansprüche

1. Vorrichtung (20; 60; 80) zum Verbreiten eines Duftstoffs, umfassend:
- zwei gegenüberstehende Wände (30; 32; 82; 84), die entlang einer Längsachse (L) derart voneinander beabstandet sind, dass sie dazwischen einen Hohlraum (C; C') definieren, der sich quer bis zu einer Peripheriewand (40) erstreckt, die mit mindestens einer Öffnung (42) für die Verbreitung eines Duftstoffs außerhalb der Vorrichtung versehen ist, wobei die mindestens eine Öffnung den Hohlraum mit dem Äußeren der Vorrichtung in Verbindung setzt,
- mindestens eine Luftwiederaufnahmemündung (O1, O2; 94a, 94b, 96a, 96b), die jede Wand durchquert, um jede externe Zone, die an jede Wand angrenzt, mit dem Hohlraum und demnach mit dem Äußeren der Vorrichtung in Verbindung zu setzen,
**dadurch gekennzeichnet, dass** sie Folgendes umfasst:
- mindestens eine Lunte (34; 86 bis 92) aus porösem Material, die sich in dem Hohlraum entlang der Längsachse erstreckt und dabei die beiden Wände über ihre jeweiligen entgegengesetzten Enden (34a, 34b) durchquert, die jeweils in eine externe Zone (Z1, Z2), die an die betreffende Wand angrenzt, vorstehen.

2. Vorrichtung nach Anspruch 1, **dadurch gekennzeichnet, dass** sie auf beiden Seiten der beiden Wände in jeder externen Zone (Z1, Z2), die an jede Wand angrenzt, Befestigungsmittel (22, 24; 104a, 104b, 106a, 106b) umfasst, die jeweils dazu gedacht sind, an der Vorrichtung einen Hals (12a, 12b; 110a, 110b) einer Flasche zu befestigen.

3. Vorrichtung nach Anspruch 1 oder 2, **dadurch gekennzeichnet, dass** jede Wand (30, 32; 82, 84) eine allgemein konkave Form aufweist, deren Konkavität zu dem Hohlraum hin orientiert ist und eine mittlere Region umfasst.

4. Vorrichtung nach einem der Ansprüche 1 bis 3, **dadurch gekennzeichnet, dass** sich die mindestens eine Mündung an der Peripherie der mittleren Region befindet.

5. Vorrichtung nach einem der Ansprüche 1 bis 4, **dadurch gekennzeichnet, dass** mehrere durchgehende Luftwiederaufnahmemündungen (O1, O2) in jeder Wand angebracht sind, wobei die Vorrichtung Verschlussmittel (62, 64) umfasst, die je nach ihrer Position geeignet sind, um die Mündungen ganz oder teilweise zu verschließen.

6. Vorrichtung nach einem der Ansprüche 1 bis 5, **dadurch gekennzeichnet, dass** sie eine Vielzahl von porösen Lunten (86 bis 92) umfasst, die in dem Hohlraum verteilt sind.

7. Vorrichtung nach einem der Ansprüche 1 bis 6, **dadurch gekennzeichnet, dass** sie ein Kugelventilsystem (100, 102) umfasst, das in jeder Wand (82, 84) rechtwinklig zu einer Mündung (100a, 102a) eingerichtet ist, welche die Wand durchquert, so dass die Kugel (100b, 102b) des Kugelventilsystems der oberen Wand die entsprechende Mündung verschließt und dass die Kugel des Kugelventilsystems der unteren Wand die entsprechende Mündung frei lässt.

8. Vorrichtung nach einem der Ansprüche 1 bis 7, **dadurch gekennzeichnet, dass** die Längsachse (L) eine senkrechte Achse der Vorrichtung ist.

9. System (10; 50; 70) zum Verbreiten eines Duftstoffs, **dadurch gekennzeichnet, dass** es Folgendes umfasst:
- eine Verbreitungsvorrichtung (20; 60; 80) nach einem der Ansprüche 1 bis 8,
- eine erste obere Flasche (12; 110), die einen ersten Hals (12a; 110a) aufweist, dessen Öffnung gegenüber einer (30; 82) der beiden Wände angeordnet ist, die der so genannten oberen Wand gegenüberstehen, eine zweite untere Flasche (14; 112), die einen zweiten Hals (14a; 112a) aufweist, dessen Öffnung gegenüber der anderen so genannten unteren Wand (32; 84) angeordnet ist, wobei die erste obere Flasche den Duftstoff umfasst, der durch Tränken der Lunte (34; 86 bis 92) einerseits die Verbreitung des Duftstoffs in dem Hohlraum (C, C') und außerhalb der Vorrichtung über die mindestens eine Öffnung (42) und andererseits das Abtropfen in die zweite untere Flasche bewirkt.

## Claims

1. Fragrance diffusion device (20; 60; 80), comprising:
- two opposing walls (30; 32; 82; 84) spaced apart from one another along a longitudinal axis (L) so as to define between them a cavity (C; C') which extends transversely as far as a peripheral wall (40) provided with at least one opening (42) for diffusing fragrance to outside the device, said at least one opening placing the cavity in communication with the outside of the device,
- at least one air intake opening (O1, O2; 94a, 94b, 96a, 96b) which passes through each wall in such a way as to place each external zone adjacent to each wall in communication with the cavity and therefore with the outside of the device, **characterized in that** it comprises,
- at least one wick (34; 86-92) made of porous material which extends in the cavity along the longitudinal axis passing through the two walls via its two respective opposite ends (34a, 34b) which each project into an external zone (Z1, Z2) adjacent to the relevant wall.

2. Device according to Claim 1, **characterized in that** it comprises, on each side of the two walls, in each external zone (Z1, Z2) adjacent to each wall, fixing means (22, 24; 104a, 104b, 106a, 106b) each intended to fix a neck (12a, 12b; 110a, 110b) of a bottle to the device.

3. Device according to Claim 1 or 2, **characterized in that** each wall (30 32; 82, 84) has a concave overall shape with the concave side facing towards the cavity and comprises a central region.

4. Device according to one of Claims 1 to 3, **characterized in that** said at least one opening is situated at the periphery of the central region.

5. Device according to one of Claims 1 to 4, **characterized in that** several air intake through-openings (O1, O2) are made in each wall, the device comprising shutoff means (62, 64) which, depending on their position, are able to shut off all or some of said openings.

6. Device according to one of Claims 1 to 5, **characterized in that** it comprises a plurality of porous wicks (86-92) distributed in the cavity.

7. Device according to one of Claims 1 to 6, **characterized in that** it comprises a ball valve system (100, 102) formed in each wall (82, 84) in line with a through-opening (100a, 102a) in the wall so that the ball (100b, 102b) of the ball valve system of the upper wall shuts off the corresponding opening and the ball of the ball valve system of the lower wall leaves the corresponding opening uncovered.

8. Device according to one of Claims 1 to 7, **characterized in that** the longitudinal axis (L) is a vertical axis of the device.

9. Fragrance diffusion system (10; 50; 70), **characterized in that** it comprises:
- a diffusion device (20; 60; 80) according to one of Claims 1 to 8,
- an upper first bottle (12; 110) having a first neck (12a; 110a) the opening of which is positioned facing one (30; 82) of the two opposing walls referred to as the upper wall; a lower second bottle (14; 112) having a second neck (14a; 112a) the opening of which is positioned facing the other wall referred to as the lower wall (32; 84), the upper first bottle comprising the fragrance which, by impregnating the wick (34; 86-92), on the one hand causes the fragrance to diffuse into the cavity (C, C') and to outside of the device via said at least one opening (42), and on the other hand causes dripping drop by drop into the lower second bottle.
